Europäisches Patentamt

European Patent Office

Office européen des brevets

(19)

(11) Veröffentlichungsnummer : **0 414 252 B1**

(12) # EUROPÄISCHE PATENTSCHRIFT

(45) Veröffentlichungstag der Patentschrift :
03.11.93 Patentblatt 93/44

(51) Int. Cl.⁵ : **C04B 28/28, A61L 27/00**

(21) Anmeldenummer : 90116237.0

(22) Anmeldetag : 24.08.90

(54) **Offenporige Formkörper, ihre Herstellung und ihre Verwendung.**

(30) Priorität : 24.08.89 DE 3927984

(43) Veröffentlichungstag der Anmeldung :
27.02.91 Patentblatt 91/09

(45) Bekanntmachung des Hinweises auf die
Patenterteilung :
03.11.93 Patentblatt 93/44

(84) Benannte Vertragsstaaten :
CH DE FR GB IT LI

(56) Entgegenhaltungen :
US-A- 3 814 717
US-A- 4 250 277

(73) Patentinhaber : THERA Patent GmbH & Co. KG
Gesellschaft für industrielle Schutzrechte
Am Griesberg 2
D-82229 Seefeld (DE)

(72) Erfinder : Jochum, Peter, Dr.
Pointweg 5
D-8031 Seefeld 2 (DE)
Erfinder : Gasser, Oswald, Dr.
Höhenstrasse 10
D-8031 Seefeld (DE)
Erfinder : Holupirek, Manfred, Dr.
Jesenwangerstrasse 2
D-8082 Grafrath (DE)
Erfinder : Wanek, Erich, Dr.
An der Breite 9
D-8031 Seefeld (DE)
Erfinder : Guggenberger, Rainer, Dr.
Sigl-Strasse 10
D-8036 Hersching (DE)
Erfinder : Stefan, Klaus-Peter, Dr.
Hauptstrasse 36a
D-8031 Seefeld (DE)
Erfinder : Ellrich, Klaus, Dr.
Auinger Strasse 16
D-8031 Wörthsee (DE)

(74) Vertreter : Abitz, Walter, Dr.-Ing. et al
Patentanwälte Abitz & Partner Postfach 86 01
09
D-81628 München (DE)

EP 0 414 252 B1

EP 0 414 252 B1

## Beschreibung

Die Erfindung betrifft offenporige Formkörper, ihre Herstellung und ihre Verwendung.

Bei chirurgischen Eingriffen sieht sich der behandelnde Arzt oft vor das Problem gestellt, natürliche Knochendefekte oder bei der Operation entstandene Knochendefekte ausgleichen zu müssen. Zum Einsatz kommen als Knochenersatzmaterial vor allem bioinerte oder bioaktive Materialien, die unter den Begriffen "Biokeramiken, Biogläser und Bioglaskeramiken" bekannt sind. Unter "bioinert" versteht man hierbei bisher Materialien, die keine Gewebereaktionen auslösen und keine Fremdstoffe abgeben. Hierzu zählen kann man auch Implantationskörper aus Titan, welche an der Oberfläche eine Schicht von Titanoxid haben, die in diesem Fall die Aufgabe der bioinerten Keramikschicht erfüllt. Als "bioaktiv" bezeichnet man nach heutigem Sprachgebrauch Materialien, die die Eigenschaft besitzen, direkt mit Knochengewebe zu verwachsen. Hierzu zählen Biogläser, Bioglaskeramiken, die an der Oberfläche deutliche Anteile von Calciumphosphatkeramiken haben, wie Hydroxylapatit- und Tricalciumphosphatkeramiken. Als Knochenersatzmaterial finden somit Anwendung verschiedene Hydroxylapatit- und Tricalciumphosphatkeramiken, die in granulierter Form sowie in vorgefertigten Formkörpern angeboten werden. Die Herstellung der Materialien erfordert recht aufwendige Sinterprozesse, bei denen über Temperatur und bestimmte Zuschlagstoffe eine Mikro- und Makroporosität erreicht werden kann.

Weiterhin ist bekannt, zur Bekämpfung von Knochendefekten Implantationsmaterialien auf Basis von Polyacrylaten (z.B. PMMA) einzusetzen, die mit Füllstoffpartikeln auf Basis Tricalciumphosphat gefüllt sind (Deutsche Offenlegungsschrift 33 25 111). In der Offenlegungsschrift wird auch beschrieben, dass es vorteilhaft sein kann, neben dem Tricalciumphosphat weitere körperverträgliche resorbierbare Stoffe zuzusetzen. Eine Porosität an der Oberfläche des Ersatzmaterials wird hierbei durch die Resorption erzeugt und soll das Einwachsen von Knochenmaterial erleichtern.

In ähnlicher Weise beschreibt die Deutsche Offenlegungsschrift 27 52 297 gefüllte Polymethylmethacrylat-Materialien, die neben einem resorbierbaren Füllstoff (z.B. $Na_2HPO_4$) Carbonate und Phosphorsäure enthalten. Durch das Zusammenwirken von Phosphorsäure und Carbonat entsteht beim Anmischen über einen Schäumungsprozess eine poröse Materialstruktur. Da die Materialien aber flüssige Monomere und Phosphorsäure enthalten, sind sie toxikologisch nicht unbedenklich. Zudem ist bei der Abbindereaktion mit hohen Temperaturen zu rechnen, wobei angrenzendes Gewebe zerstört werden kann. Eine "bioaktive" Wirkung ist angesichts dieser Tatsache wohl kaum möglich.

Im Bereich der Zahnmedizin werden seit längerer Zeit sogenannte "Glasionomerzemente" eingesetzt. Hier handelt es sich um Reaktionsprodukte aus einem Aluminiumfluorosilikatglaspulver mit einer wasserlöslichen Polycarbonsäure und Wasser. Diese Materialien werden vor allem als Zahnfüllmaterial, Zahnzementmaterial angewendet, auch der Einsatz als Knochenzement, also zum Befestigen von Prothesen im Knochen wurde beschrieben (siehe z.B. Deutsche Offenlegungsschrift 29 29 121).

Die DE-OS 27 36 447 beschreibt ein Verfahren zur Herstellung von Polymerkeramikmaterialien aus einem Ionen-laugbaren Glaspulver und einer Polycarbonsäure, bei dem die Reaktionskomponenten in der Weise auf eine Substratoberfläche gesprüht werden, dass die Komponenten erst nach Verlassen des Sprühkopfes miteinander umgesetzt werden. Den beiden Reaktionskomponenten kann ein Blähmittel zugesetzt werden, um eine Verschäumung zu erreichen. Die nach diesem Sprühverfahren erreichbare Verschäumung ist für die vorliegenden Zwecke nicht zufriedenstellend, da das erzielbare Gesamtporenvolumen (Vp) zu gering ist. Für die erfindungsgemässen Zwecke ist es erforderlich, dass offenporige Formkörper hergestellt werden, deren Poren untereinander verbunden sind und ein Gesamtporenvolumen (Vp) von mindestens 30 Vol.-% aufweisen, so dass bei Einsatz der Formkörper als Knochenersatzmaterial das Knochenmaterial problemlos und in ausreichendem Ausmass durch die Formkörper hindurchwachsen kann. Die in der Offenlegungsschrift beschriebenen Materialien dienen in erster Linie als Baumaterialien, z.B. Isolierbauziegel (siehe Seite 6 oben).

In der älteren EP-A-331071 (entsprechend der deutschen Offenlegungsschrift P 38 06 448) werden poröse Formkörper aus einem verformbaren Material beschrieben, welches keine toxikologisch bedenklichen, niedermolekularen Monomeren enthält und bioaktiv ist. Die porösen Formkörper enthalten das Produkt der Verschäumung und Härtung von (a) Aluminiumfluorosilikatglas, (b) mindestens einer Polycarbonsäure mit einem mittleren Molekulargewicht > 500, (c) einem Carbonat und/oder Hydrogencarbonat als Verschäumungsmittel, (d) gegebenenfalls einem chelatbildenden Mittel und (e) Wasser, und eignen sich als Knochenersatzmaterial.

Die vorliegende Erfindung betrifft eine Weiterentwicklung dieser Formkörper.

Gegenstand der Erfindung sind offenporige Formkörper, enthaltend das Produkt der Verschäumung und Härtung von (a) einem Aluminiumfluorosilikatglas, (b) mindestens einer Polycarbonsäure mit einem mittleren Molekulargewicht > 500, (c) einem Verschäumungsmittel, ausgenommen Carbonate und/oder Hydrogencarbonate, (d) gegebenenfalls einem Chelatisierungsmittel und (e) Wasser, wobei das Gesamtporenvolumen (Vp) der Formkörper 30 bis 70 Vol-% beträgt.

2

Die erfindungsgemässen offenporigen Formkörper eignen sich insbesondere als Knochenersatzmaterial. Die Poren sind untereinander verbunden.

Vorzugsweise liegen die erfindungsgemässen offenporigen Formkörper in Form von Granulaten, Quadern oder anatomisch geformten Körpern als Knochenersatzmaterial vor.

Das Gesamtporenvolumen (Vp) der erfindungsgemässen offenporigen Formkörper beträgt vorzugsweise 40 bis 60 Vol.-%.

Gegenstand der Erfindung ist weiterhin die Verwendung von offenporigen Formkörpern, enthaltend das Produkt der Verschäumung und Härtung von

(a) einem Aluminiumfluorosilikatglas,

(b) mindestens einer Polycarbonsäure mit einem mittleren Molekulargewicht > 500,

(c) einem Verschäumungsmittel, ausgenommen Carbonat und/oder Hydrogencarbonat,

(d) gegebenenfalls einem chelatbildenden Mittel und

(e) Wasser

zur Herstellung von implantierbaren Materialien, sowie die Verwendung von offenporigen Formkörpern, erhalten durch Verschäumen und Härten von

(a) einem Aluminiumfluorosilikatglas,

(b) mindestens einer Polycarbonsäure mit einem mittleren Molekulargewicht > 500,

(c) einem Verschäumungsmittel, ausgenommen Carbonate und/oder Hydrogencarbonate,

(d) gegebenenfalls einem chelatbildenden Mittel und

(e) Wasser

zur Herstellung von Granulaten, Quadern oder anatomisch geformten Körpern als knochenähnliche Implantate.

Die Herstellung der erfindungsgemässen offenporigen Formkörper kann dadurch erfolgen, dass man

(a) ein Aluminiumfluorosilikatglas,

(b) mindestens eine Polycarbonsäure mit einem mittleren Molekulargewicht > 500,

(c) ein Verschäumungsmittel, ausgenommen Carbonate und/oder Hydrogencarbonate,

(d) gegebenenfalls ein chelatbildendes Mittel und

(e) Wasser

homogen miteinander vermischt (nicht versprüht) und unter gleichzeitiger und/oder nachfolgender Formgebung verschäumen und härten lässt.

Als Verschäumungsmittel (c) eignen sich alle Materialien, die dazu geeignet sind, beim Herstellen der Formkörper eine Verschäumung zu bewirken. So ist es z.B. möglich, mit den flüssigen Bestandteilen ($H_2O$ oder $H_2O$ + Säure) unter Zusatz von Tensiden und/oder Emulgatoren und Einrühren von Gasen (z.B. Luft) stabile Schäume zu erzeugen, zu denen dann die anderen Bestandteile (z.B. Glas) hinzugefügt werden (siehe z.B. Ullmanns Enzyklopädie der technischen Chemie, Band 22, S. 463, Verlag Chemie, 4. Auflage, 1982). Weiter sind geeignet metallische Hydride, insbesondere Natriumborhydrid, welche mit Protonen (Wasser oder Säuren) unter Wasserstoffentwicklung eine Aufschäumung des härtenden Zements bewirken.

Eine weitere Möglichkeit der Schaumbildung besteht darin, eine wässrige Lösung eines Gases zu verwenden, z.B. von $CO_2$ oder $SO_2$, welches durch die zugegebene Säure beim Herstellen des Zements ausgetrieben wird und somit eine Schäumung bewirkt.

Möglich ist auch der Zusatz von Peroxiden, z.B. $H_2O_2$, die entweder durch Säureeinwirkung oder aber durch Metallkatalyse unter Zersetzung und Sauerstoffabgabe zu einem Verschäumen führen. So ist es beispielsweise möglich, eine wässrige $H_2O_2$-Lösung zum Anmischen des Zementes zu verwenden und in das Pulver eine entsprechende Menge $FeSO_4$ einzugeben, welches dann beim Zusammenbringen der beiden Komponenten zu einer Sauerstoffentwicklung und somit zu einer Aufschäumung führt.

Weitere Verschäumungsmittel sind Treibmittel, wie feste organische Treibmittel, z.B. Azodicarbonamid, Azobis-(isobutyronitril), Diphenyloxid-disulfonsäurehydrazid oder N-Nitroso-Verbindungen, feste anorganische Treibmittel, flüssige Treibmittel, z.b. Kohlenwasserstoffe oder Halogenkohlenwasserstoffe, und gasförmige Treibmittel, wie $N_2$, $CO_2$ und Luft.

Feste und flüssige Verschäumungsmittel werden üblicherweise in einer Konzentration von 0,01 - 50 Gew.-%, bezogen auf die Gesamtmischung, besonders bevorzugt 0,1 - 20 Gew.-%, eingesetzt, gasförmige Verschäumungsmittel werden in Mengen von 5 - 90 Vol.-%, bezogen auf die Gesamtmischung, vorzugsweise 10 - 60 Vol.-%, eingesetzt.

Das Verschäumungsmittel kann zunächst eine bis drei der Komponenten (a), (b), (d) und (e) verschäumen, wobei danach die restlichen Bestandteile zugefügt werden. Es können aber auch Vormischungen der Bestandteile gebildet werden, von denen eine das Verschäumungsmittel enthält, die erst bei ihrem Zusammengeben verschäumen. Wenn das Verschäumungsmittel aus zwei Bestandteilen besteht, die beim Zusammengeben eine Verschäumung bewirken, so sind diese Bestandteile in getrennten Vormischungen enthalten.

Das Verschäumungsmittel kann aber auch erst zu der Mischung aller anderen Bestandteile hinzugegeben werden.

Zusätzlich können die erfindungsgemäss eingesetzten Ausgangsmaterialien Konservierungsmittel, wie Benzoesäure, sowie Thixotropiehilfsmittel, Füllstoffe oder Pigmente enthalten.

Die Herstellung der Formkörper kann durch Verschäumen und Einbringen des Zements in Formen mit möglichst kleinem Oberflächen/Volumen-Verhältnis erfolgen. Während der Aushärtung kann dabei eine Volumenvergrösserung aufgrund der Schäumung beobachtet werden. Während sich der Zement in einer plastischen Phase befindet, kann die kompakte Oberfläche abgeschält werden. Die porösen Formkörper weisen damit nach aussen offene Poren auf. Die vollständige Aushärtung kann durch Stehenlassen bei 36°C/100% relative Luftfeuchtigkeit erfolgen. Der ausgehärtete Zement kann zur Herstellung von Granulat auf die entsprechende Kornfraktion gemahlen und gesiebt werden.

Zur Auffüllung von Knochendefekten eignen sich z.B. erfindungsgemässe Granulate, die beispielsweise eine Korngrösse von 0,2 - 5 mm aufweisen, besonders bevorzugte Korngrössenfraktionen sind von 0,2 - 0,5 mm, von 0,5 - 1 mm, von 1 - 2 mm, von 2 - 3 mm, von 3 - 4 mm und von 4 - 5 mm.

Da es bekannt ist [J.F. Osborne, Zahnärztliche Mitteilungen 77, 840 (1987)], dass das Einwachsen von Knochen durch scharfe Kanten verhindert wird und diese auch gleichzeitig das Entzündungsrisiko erhöhen, wird das Granulat nach der Fraktionierung vorzugsweise verrundet. Das Verrunden erfolgt beispielsweise durch 24- bis 48-stündiges Rollen in $H_2O$ (destilliert) oder in verdünnter Magnesiumchloridlösung. Nach der Verrundung kann das Granulat dann auf einem der Korngrösse entsprechenden Sieb abfiltriert und frei von Splitter und Feinanteil gewaschen werden.

Die erhaltenen Formkörper, z.B. Granulate, können anschliessend autoklaviert werden, z.B. durch 20-minütiges Erhitzen auf 121°C.

Zur Verblockung in der Wirbelsäulenchirurgie oder in der Knochenchirurgie (Traumatologie) und zur Überbrückung langstreckiger Knochendefekte können auch Formkörper in Form von Quadern unterschiedlicher Kantenlänge eingesetzt werden. Anatomisch geformte poröse Körper eignen sich besonders für die plastische Chirurgie, z.B. als Jochbeinersatz oder zum Kinnaufbau.

Die Porosität der Formkörper lässt sich in an sich bekannter Weise durch das Gesamtporenvolumen ausdrücken. Die Bestimmung des Gesamtporenvolumens kann z.B. aufgrund der Dichteunterschiede von geschäumtem und nicht geschäumtem Material bestimmt werden. So erhält man das Gesamtporenvolumen Vp (ausgedrückt als Volumen-%) nach Formel I:

$$Vp = \frac{100 \times (\rho k - \rho g)}{\rho k} \quad \text{Formel I}$$

$\rho k$ ist hierbei die Dichte des kompakten Materials,
$\rho g$ ist die Dichte des geschäumten Materials.

Eine weitere Methode zur Bestimmung des Gesamtporenvolumens besteht in der Quecksilberporosimetrie. Hierbei wird mit steigendem Druck Quecksilber in die Poren gepresst. Der Druck korreliert mit der Porengrösse, das bei einem bestimmten Druck eingepresste Quecksilbervolumen entspricht dem Gesamtvolumen der Poren dieser Grösse. Das Gesamtporenvolumen (Vp) ergibt sich somit nach Formel II:

$$Vp = \frac{V_{Hg}}{Vg} \times 100 \% \quad \text{Formel II}$$

Mit der Methode der Quecksilberporosimetrie lassen sich insbesondere die porösen Granulate auf ihre Porosität bestimmen.

Als Bestandteil (a) können die in der Deutschen Offenlegungsschrift 20 61 513 und der Europäischen Patentveröffentlichung 0 023 013 beschriebenen Calciumaluminiumfluorosilikatgläser und die in der Europäischen Patentveröffentlichung 0 241 277 beschriebenen Strontiumaluminiumfluorosilikatgläser eingesetzt werden. Die erfindungsgemäss verwendeten Aluminiumfluorosilikatglas-Pulver bestehen neben Sauerstoff vorzugsweise aus:

| Bestandteil | Berechnet als | Gew.-% |
|---|---|---|
| Si | $SiO_2$ | 20 - 60 |
| Al | $Al_2O_3$ | 10 - 50 |
| Ca | CaO | 0 - 40 |
| Sr | SrO | 0 - 40 |
| F | F | 1 - 40 |
| Na | $Na_2O$ | 0 - 10 |
| P | $P_2O_5$ | 0 - 10 |

wobei mindestens 1 Gew.-% CaO und/oder SrO enthalten sein müssen und insgesamt 0 bis 20 Gew.-%, berechnet als Oxide, an B, Bi, Zn, Mg, Sn, Ti, Zr, La oder anderen 3-wertigen Lanthanoiden, K, W, Ge sowie weiteren Zusätzen, welche die Eigenschaften nicht beeinträchtigen und physiologisch unbedenklich sind. Durch Zusatz von 10 bis 20 Gew.-% $La_2O_3$ können die Gläser röntgensichtbar gemacht werden.

Vorteilhaft bestehen die Pulverteilchen aus:

| | | |
|---|---|---|
| Si als $SiO_2$ | 25 - 50 | Gew.-% |
| Al als $Al_2O_3$ | 10 - 40 | Gew.-% |
| Ca als CaO | 0 - 35 | Gew.-% |
| Sr als SrO | 0 - 35 | Gew.-% |
| F | 5 - 30 | Gew.-% |
| Na als $Na_2O$ | 0 - 8 | Gew.-% |
| P als $P_2O_5$ | 1 - 10 | Gew.-% |

wobei mindestens 10 Gew.-% Ca (berechnet als CaO) und/oder Sr (berechnet als SrO) enthalten sein müssen und 0 - 10 Gew.-% an $B_2O_3$, $Bi_2O_3$, ZnO, MgO, $SnO_2$, $TiO_2$, $ZrO_2$, $La_2O_3$ oder anderen Oxiden 3-wertiger Lanthanoide, $K_2O$, $WO_3$, $GeO_2$ sowie weiteren Zusätzen, welche die Eigenschaften nicht beeinträchtigen und physiologisch unbedenklich sind.

Besonders bevorzugt verwendete Pulver enthalten

| | | |
|---|---|---|
| Si als $SiO_2$ | 25 - 45 | Gew.-% |
| Al als $Al_2O_3$ | 20 - 40 | Gew.-% |
| Ca als CaO | 10 - 30 | Gew.-% |
| F | 10 - 30 | Gew.-% |
| Na als $Na_2O$ | 1 - 8 | Gew.-% |
| P als $P_2O_5$ | 1 - 10 | Gew.-% |

Die erfindungsgemäss verwendeten Glaspulver weisen eine durchschnittliche Korngrösse (Gewichtsmittel) von wenigstens 1 μm und bevorzugt mindestens 3 μm auf. Die durchschnittliche Korngrösse (Gewichtsmittel) beträgt 1 - 20 μm, bevorzugt 3 - 15 μm und besonders bevorzugt 3 - 10 μm. Die Teilchen haben eine maximale Korngrösse von 150 μm, vorzugsweise 100 μm, besonders 60 μm.

Die so erhaltenen Pulver werden dann gegebenenfalls einer Oberflächenbehandlung gemäss der europäischen Patentschrift 0 023 013 unterzogen. Hierzu werden die Glaspulver mit Säure oberflächlich behandelt, vorzugsweise bei Raumtemperatur. Dabei werden saure Gruppen enthaltende Substanzen eingesetzt, z.B. Salzsäure, Schwefelsäure, Salpetersäure, Essigsäure, Propionsäure oder Perchlorsäure, die lösliche Calciumsalze bzw. Strontiumsalze bilden. Die Säuren werden in einer Konzentration von 0,01 bis 10 Gew.-%, vorzugsweise von 0,05 bis 3 Gew.-% eingesetzt. Nach der entsprechenden Reaktionszeit werden die Pulver von der Lösung separiert und gründlich ausgewaschen, so dass sich praktisch keine löslichen Calcium- oder Strontiumsalze mehr auf der Oberfläche der Pulverteilchen befinden.

Die als Bestandteil (b) einzusetzenden Polycarbonsäuren können auch die bei der Herstellung von Glasionomerzementpulvern bekannten Polycarbonsäuren, z.B. Polymaleinsäure, Polyacrylsäure, Polyitaconsäure sowie Gemische davon oder Copolymere, insbesondere die aus der Europäischen Patentschrift 0 024 056 bekannten Maleinsäure-Acrylsäure-Copolymeren und/oder Acrylsäure-Itakonsäure-Copolymeren sein. Das mittlere Molekulargewicht der erfindungsgemäss einzusetzenden Polycarbonsäuren beträgt mehr als 500. Vorteilhaft ist ein mittleres Molekulargewicht von 1.000 bis 20.000, besonders bevorzugt sind 3.000 bis 10.000. Die Polycarbonsäure wird vorzugsweise in Konzentrationen von 5 bis 50 Gew.-%, bezogen auf Bestandteil (a), eingesetzt.

Als Bestandteil (d) kann ein chelatbildendes Mittel, wie es in der Deutschen Offenlegungsschrift 23 19 715 beschrieben ist, enthalten sein. Vorzugsweise wird als Chelatbildner Weinsäure eingesetzt.

Mit den erfindungsgemässen offenporigen Formkörpern werden gut verträgliche Knochenersatzteile erhalten, die aufgrund ihrer offenen Porosität vom Knochenmaterial gut durchwachsen werden können.

## BEISPIELE

In allen Beispielen wird ein Calciumaluminiumfluorosilikatglaspulver mit der in Tabelle 1 wiedergegebenen oxidischen Zusammensetzung eingesetzt.

## Tabelle 1

|  | Gew.-% |
|---|---|
| Si als $SiO_2$ | 33,8 |
| Al als $Al_2O_3$ | 28,3 |
| Ca als CaO | 14,4 |
| Na als $Na_2O$ | 2,6 |
| P als $P_2O_5$ | 6,7 |
| F | 17,3 |

Die durchschnittliche Teilchengrösse liegt bei 5 μm.

### Beispiel 1

100 Gew.-teile des Glaspulvers werden mit 0,5 Gew.-teilen Natriumborhydrid (Fa. Merck) zu einem homogenen Pulver vermischt. 2 Gew.-teile dieses Pulvers werden mit einer Lösung, bestehend aus 35% eines Copolymeren aus Acrylsäure und Maleinsäure (1:1, durchschnittliches Molekulargewicht 7000), und 65% $H_2O$ dest. zu einer homogenen Paste angemischt. Das Material hat eine Verarbeitungszeit von ca. 4 Minuten, 30 Sekunden (gerechnet vom Anmischbeginn) und wird nach 12 - 13 Minuten vollständig hart. Das Material schäumt dabei zu einem porösen Formkörper mit Porengrössen zwischen 0,1 und 3 mm. Die Charakterisierung des porösen Materials wird in Tabelle 2 wiedergegeben.

### Beispiel 2

100 Gew.-teile des Calciumaluminiumfluorosilikatglaspulvers werden mit 18 Gew.-teilen Eisen(II)sulfat (Fa. Merck) sowie 20,4 Gew.-teilen des Copolymers aus Beispiel 1 zu einem homogenen Pulver vermischt. (Das trockene Copolymer wird mit 0,9 Gew.-% Benzoesäure, bezogen auf das Copolymer, stabilisiert).

3,4 Gew.-teile dieser so erhaltenen Pulvermischung werden mit 1 Gew.-teil einer 30%igen $H_2O_2$-Lösung (Fa. Merck) homogen innerhalb einer halben Minute vermischt. Während der Verarbeitungszeit von ca. 5 Minuten (gerechnet vom Anmischbeginn) schäumt das Material auf und wird nach ca. 15 Minuten zu einem festen, geschäumten Formkörper, mit Porengrössen zwischen 0,1 und 1 mm. Die Charakterisierung des porösen Materials ist in Tabelle 2 wiedergegeben.

### Beispiel 3

100 Gew.-teile des Calciumaluminiumfluorosilikatglaspulvers werden mit 3,8 Gew.-teilen Kupfersulfat (Fa. Merck), 1,9 Gew.-teilen Aluminiumpulver (Fa. Merck) sowie 20,3 Gew.-teilen des Copolymers aus Beispiel 1 zu einem homogenen Pulver vermischt. (Das trockene Copolymer wird mit 0,9 Gew.-% Benzoesäure, bezogen auf das Copolymer, stabilisiert).

3,4 Gew.-teile dieser so erhaltenen Pulvermischung werden mit 1 Gew.-teil einer 30%igen $H_2O_2$-Lösung (Fa. Merck) homogen innerhalb einer halben Minute vermischt. Während der Verarbeitungszeit von ca. 5 Minuten (gerechnet vom Anmischbeginn) schäumt das Material auf und wird nach ca. 15 Minuten zu einem festen, geschäumten Formkörper.

### Beispiel 4

Aus den porösen Materialen der Beispiele 1 - 3 wird durch Zerstossen und anschliessendes Sieben ein Granulat der Korngrösse 0,5 - 1 mm hergestellt. Von diesen Granulaten werden die Schüttdichte und die spezifische Oberfläche (nach Brunauer, Emmett und Teller, wobei Stickstoff adsorbiert und die dabei beobachtete Volumenänderung gemessen wird - BET) bestimmt. Die Ergebnisse sind in Tabelle 2 zusammengefasst.

### Beispiel 5

Es wurden, wie in Beispiel 1 beschrieben, Formkörper jedoch ohne Schäumungsmittel (NaBH$_4$) hergestellt. Die Werte sind Tabelle 2 zu entnehmen.

## Tabelle 2

| Beispiel | Verschäumungsmittel | Dichte der Formkörper [g/cm³] | Gesamtporenvol. [%] | Interkonnektierendes Porensystem | Granulat 0,5-1 mm (Beispiel 5) | |
|---|---|---|---|---|---|---|
| | | | | | Schüttdichte [g/cm³] | spezifische Oberfläche [m²/g] |
| 1 | $NaBH_4$ | 0,7 | 65 | ja | 0,756 | 78 |
| 2 | $FeSO_4/H_2O_2$ | 1,34 | 33 | ja | 0,688 | 88 |
| 3 | $CuSO_4/Al/H_2O_2$ | 1,05 | 47,5 | ja | 0,551 | 37 |
| 5 (Vergleich) | --- | 2,0 | 0 | nein | | |

EP 0 414 252 B1

EP 0 414 252 B1

**Patentansprüche**

1. Offenporige Formkörper, enthaltend das Produkt der Verschäumung und Härtung von
   (a) einem Aluminiumfluorosilikatglas,
   (b) mindestens einer Polycarbonsäure mit einem mittleren Molekulargewicht > 500,
   (c) einem Verschäumungsmittel, ausgenommen Carbonat und/oder Hydrogencarbonat,
   (d) gegebenenfalls einem chelatbildenden Mittel und
   (e) Wasser,
   wobei das Gesamtporenvolumen (Vp) der Formkörper 30 bis 70 Vol.-% beträgt.

2. Formkörper nach Anspruch 1 in Form von Granulaten, Quadern oder anatomisch geformten Körpern als Knochenersatzmaterial.

3. Formkörper nach Anspruch 1 oder 2, dadurch gekennzeichnet, dass das Gesamtporenvolumen (Vp) 40 bis 60 Vol.-% beträgt.

4. Verfahren zur Herstellung der offenporigen Formkörper nach den Ansprüchen 1 bis 3, dadurch gekennzeichnet, dass man
   (a) ein Aluminiumfluorosilikatglas,
   (b) mindestens eine Polycarbonsäure mit einem mittleren Molekulargewicht > 500,
   (c) ein Verschäumungsmittel, ausgenommen Carbonate und/oder Hydrogencarbonate,
   (d) gegebenenfalls ein chelatbildendes Mittel und
   (e) Wasser
   homogen miteinander vermischt (nicht versprüht) und unter gleichzeitiger und/oder nachfolgender Formgebung verschäumen und härten lässt.

5. Verwendung der Formkörper nach den Ansprüchen 1 bis zur Herstellung von implantierbaren Materialien.

6. Verwendung von offenporigen Formkörpern, enthaltend das Produkt der Verschäumung und Härtung von
   (a) einem Aluminiumfluorosilikatglas,
   (b) mindestens einer Polycarbonsäure mit einem mittleren Molekulargewicht > 500,
   (c) einem Verschäumungsmittel, ausgenommen Carbonat und/oder Hydrogencarbonat,
   (d) gegebenenfalls einem chelatbildenden Mittel und
   (e) Wasser,
   zur Herstellung von implantierbaren Materialien.

7. Verwendung von offenporigen Formkörpern, erhalten durch Verschäumen und Härten von
   (a) einem Aluminiumfluorosilikatglas,
   (b) mindestens einer Polycarbonsäure mit einem mittleren Molekulargewicht > 500,
   (c) einem Verschäumungsmittel, ausgenommen Carbonat und/oder Hydrogencarbonat,
   (d) gegebenenfalls einem chelatbildenden Mittel und
   (e) Wasser,
   in Form von Granulaten, Quadern oder anatomisch geformten Körpern als knochenähnliche Implantate.

**Claims**

1. Open-pored mouldings containing the product of the foaming and setting of
   (a) an aluminium fluorosilicate glass,
   (b) at least one polycarboxylic acid with an average molecular weight > 500,
   (c) a foaming agent, other than carbonates and/or hydrogencarbonates,
   (d) optionally a chelating agent and
   (c) water,
   in which the total pore volume ($V_p$) of the mouldings is 30 to 70% by volume.

2. Mouldings according to claim 1 in the form of granules, parallelepipeds or anatomically shaped bodies as bone substitute material.

9

3.  Mouldings according to claim 1 or 2, characterized in that the total pore volume ($V_p$) is 40 to 60 % by volume.

4.  Process for the production of the open-pored mouldings according to claims 1 to 3, characterized in that
    (a) an aluminium fluorosilicate glass,
    (b) at least one polycarboxylic acid with and average molecular weight >500,
    (c) a foaming agent, other than carbonates and/or hydrogencarbonates,
    (d) optionally a chelating agent and
    (e) water

    are homogeneously mixed together (not sprayed) and foamed with simultaneous and/or subsequent forming and then set.

5.  Use of the mouldings according to claims 1 to 3 for the production of implantable materials.

6.  Use of open-pored mouldings, containing the product of the foaming and setting of
    (a) an aluminium fluorosilicate glass,
    (b) at least one polycarboxylic acid with and average molecular weight > 500,
    (c) a foaming agent, other than carbonates and/or hydrogencarbonates,
    (d) optionally a chelating agent and
    (e) water
    for the production of implantable materials.

7.  Use of open-pored mouldings, obtained by the foaming and setting of
    (a) an aluminium fluorosilicate glass,
    (b) at least one polycarboxylic acid with and average molecular weight > 500,
    (c) a foaming agent, other than carbonates and/or hydrogencarbonates,
    (d) optionally a chelating agent and
    (e) water
    in the form of granules, parallelepipeds or anatomically shaped bodies, as bone-like implants.

**Revendications**

1.  Corps moulés à pores ouverts, contenant le produit de la formation de mousse et du durcissement :
    (a) d'un verre de fluorosilicate d'aluminium,
    (b) d'au moins un acide polycarboxylique ayant un poids moléculaire moyen > 500,
    (c) d'un agent de formation de mousse, à l'exclusion d'un carbonate et/ou d'un hydrocarbonate,
    (d) éventuellement d'un agent de chélation, et
    (e) d'eau,
        le volume total des pores (Vp) des corps moulés étant compris entre 30 et 70 % en volume.

2.  Corps moulés suivant la revendication 1, se présentant sous la forme de granulés, de dés ou de corps ayant reçu un forme anatomique, servant de matériau de remplacement des os.

3.  Corps moulés suivant la revendication 1 ou 2, caractérisés en ce que que le volume total des pores (Vp) est compris entre 40 et 60 % en volume.

4.  Procédé de fabrication des corps moulés, à pores ouverts, suivant les revendications 1 à 3, caractérisé en ce qu'on mélange d'une manière homogène entre eux (on ne les pulvérise pas)
    (a) un verre de fluorosilicate d'aluminium,
    (b) au moins un acide polycarboxylique ayant un poids moléculaire moyen > 500,
    (c) un agent de formation de mousse, à l'exclusion d'un carbonate et/ou d'un hydrocarbonate,
    (d) éventuellement un agent de formation d'un chélate et
    (e) de l'eau,
    et on laisse s'effectuer une formation de mousse et un durcissement avec une mise en forme simultanée et/ou ultérieure.

5.  Utilisation des corps moulés suivant les revendications 1 à 3, pour la fabrication de matériaux pouvant être implantés.

6. Utilisation de corps moulés à pores ouverts, contenant le produit de la formation de mousse et du durcissement :

   (a) d'un verre de fluorosilicate d'aluminium,
   (b) d'au moins un acide polycarboxylique ayant un poids moléculaire moyen > 500,
   (c) d'un agent de formation de mousse, à l'exclusion d'un carbonate et/ou d'un hydrocarbonate,
   (d) éventuellement d'un agent de formation d'un chélate, et
   (e) d'eau,

   en vue de la préparation de matériaux pouvant être implantés.

7. Utilisation de corps moulés à pores ouverts, obtenus par la formation de mousse et le durcissement :

   (a) d'un verre de fluorosilicate d'aluminium,
   (b) d'au moins un acide polycarboxylique ayant un poids moléculaire moyen > 500,
   (c) d'un agent de formation de mousse, à l'exclusion d'un carbonate et/ou d'un hydrocarbonate,
   (d) éventuellement d'un agent de formation d'un chélate, et
   (e) d'eau,

   sous la forme de granulés, dés ou corps ayant une forme anatomique, servant d'implants analogues à des os.